# EUROPEAN PATENT SPECIFICATION

(11) **EP 3 682 894 B1**
(45) Date of publication and mention of the grant of the patent: **17.11.2021**
(21) Application number: 18866413.0
(22) Date of filing: 22.01.2018
(51) Int. Cl.: A61K 38/12, A61K 9/19, A61K 9/107, A61K 47/34, A61P 35/00, A61P 1/00, A61K 31/704, A61P 1/18, A61P 11/00, C07K 7/06

(54) **CELL AUTOPHAGY INHIBITOR AND PREPARATION METHOD THEREFOR AND APPLICATION THEREOF**
ZELLAUTOPHAGIE-INHIBITOR UND HERSTELLUNGSVERFAHREN DAFÜR UND ANWENDUNG DAVON
INHIBITEUR D'AUTOPHAGIE CELLULAIRE, SON PROCÉDÉ DE PRÉPARATION ET SON APPLICATION

(30) Priority: 12.10.2017 CN 201710947402
(43) Date of publication of application: 22.07.2020
(73) Proprietor: China Pharmaceutical University, Nanjing, Jiangsu 211198 (CN); Sichuan University, Chengdu, Sichuan 610041 (CN)
(72) Inventor: TAN, Ninghua, Nanjing Jiangsu 211198 (CN); CHEN, Lijuan, Chengdu Sichuan 610041 (CN); SONG, Lihua, Nanjing Jiangsu 211198 (CN); YANG, Jianhong, Chengdu Sichuan 610041 (CN); WANG, Zhe, Nanjing Jiangsu 211198 (CN)
(74) Representative: Hanna Moore + Curley
(86) International application number: PCT/CN2018/073572
(87) International publication number: WO 2019/071875

(56) References cited:
- CN-A- 103 877 562
- CN-A- 104 262 465
- CN-A- 107 496 901
- HOI-WING LEUNG ET AL: "RA-XII inhibits tumour growth and metastasis in breast tumour-bearing mice via reducing cell adhesion and invasion and promoting matrix degradation", SCIENTIFIC REPORTS, vol. 5, no. 1, 23 November 2015 (2015-11-23), pages 1-17, XP055616419, DOI: 10.1038/srep16985
- CHRIS OERLEMANS ET AL: "Polymeric Micelles in Anticancer Therapy: Targeting, Imaging and Triggered Release", PHARMACEUTICAL RESEARCH, KLUWER ACADEMIC PUBLISHERS-PLENUM PUBLISHERS, NL, vol. 27, no. 12, 20 August 2010 (2010-08-20), pages 2569-2589, XP019862797, ISSN: 1573-904X, DOI: 10.1007/S11095-010-0233-4
- LIHUA SONG ET AL: "Natural Cyclopeptide RA-XII, a New Autophagy Inhibitor, Suppresses Protective Autophagy for Enhancing Apoptosis through AMPK/mTOR/P70S6K Pathways in HepG2 Cells", MOLECULES ONLINE, vol. 22, no. 11, 11 November 2017 (2017-11-11), page 1934, XP055718181, DE ISSN: 1433-1373, DOI: 10.3390/molecules22111934
- ITOKAWA, HIDEJI: "Studies on the antitumor cyclic hexapeptides obtained from Rubiae radix", Chem. Pharm. Bull., vol. 31, no. 4, 30 April 1983 (1983-04-30) , pages 1424-1427, XP002141431, ISSN: 1347-5223
- FAN, JUN-TING: "Rubiyunnanins C -H, cytotoxic cyclic hexapeptides from Rubia yunnanensis inhibiting nitric oxide production and NF-kappa B activation", Bioorganic & Medicinal Chemistry, vol. 18, no. 23, 31 October 2010 (2010-10-31), XP027546679, ISSN: 0968-0896

## Description

### Technical field

The present invention belongs to medical technology, and particularly relates to a cell autophagy inhibitor using a rubiaceae-type cyclopeptide as the active ingredient, and a preparation method and a use thereof.

### Background

KRAS gene is a signaling protein in the "downstream region" of the intracellular signaling pathway, which has an important effect on cell growth, survival, and differentiation. Under normal physiological conditions, cells activate signaling pathways such as EGFR after being stimulated by the outside world, and wild type KRAS is transiently activated after being phosphorylated by tyrosine kinases such as active EGFR, and the activated KRAS can activate the signaling proteins in the downstream of the signaling pathway, and then KRAS is rapidly inactivated, in which KRAS activation/inactivation effects are controlled. Mutant KRAS protein causes protein dysfunction, which is still activated in the absence of EGFR activation signal stimulation and its functional state is uncontrollable, leading to continuous proliferation of tumor cells, such as KRAS mutant HCT116 cells. KRAS mutants are further divided into a KRAS dependent type such as H441 and H358 cells and a KRAS independent type such as A549 and H460 cells, wherein the growth and survival of KRAS dependent tumor cells are completely dependent on the KRAS gene. In many human cancers, the KRAS gene is frequently mutated in human malignant tumors, including colon cancer, rectal cancer, lung cancer, and pancreatic cancer, making related cancers difficult to treat. Targeted drug therapy for KRAS gene activation mutations has become an excellent choice for medical workers, unfortunately, there is still no clinically effective drug strategy for treating tumors with KRAS gene mutations. In recent years, through RNAi screening of the complete genome, international leading research groups have discovered multiple genes that have a synergistic lethal relationship with the oncogene KRAS, and TAK1 is one of them. Inhibiting TAK1 activity can induce apoptosis of KRAS dependent cells, the activity of TAK1 is important to maintain the survival of KRAS dependent cells, therefore, the application of TAK1 inhibitors can selectively inhibit the survival of KRAS dependent tumor cells and provide a new targeted therapeutic strategy for KRAS dependent tumor cells.

Cell autophagy is a process of cell catabolism, which plays an important role in cell survival under the conditions of cell growth, differentiation, homeostasis and nutritional deficiencies, and is an important self-protection and defense mechanism of the body. Cell autophagy specifically refers: by enveloping part of the cytoplasm, damaged proteins, and senescent or damaged organelles such as mitochondria, Golgi apparatus, endoplasmic reticulum, etc. to form a autophagosome, which is delivered to the lysosome and fused with it to form an autophagy lysosome, and the content is digested and degraded by proteolytic enzymes to meet the metabolism and renewal of the cells. KRAS dependent tumor cells have higher basal autophagy, which is necessary for tumor cell survival under hunger and tumorigenic conditions. In KRAS dependent tumor cells, activating TAK1 so as to activate the TAK-AMPK signaling pathway and enhance the tumor cell basal autophagy and protect cells from TRAIL-induced cell death.

Polymer micelles are one of the important contents in the research of nano-systems, which have become the research focus in recent years. Polymer micelles include two parts, a drug-loaded hydrophobic core and a hydrophilic shell. The amphiphilic block polymer includes hydrophobic segments and hydrophilic segments, when the concentration in an aqueous solution exceeds the critical micelle concentration, the hydrophobic segments are close to each other to form a hydrophobic core, the hydrophilic segments face outward to form a hydrophilic shell, and the hydrophobic core and hydrophilic shell form micelles spontaneously. Hydrophobic drugs are encapsulated in the hydrophobic core by means of water repellency or covalent bonding, therefore, polymer micelles, as carriers of poorly water-soluble drugs, show great advantages and potential in drug delivery. Polymer micelles can deliver various types of drugs, including low molecular weight anticancer drugs, imaging agents, proteins, plasmid DNA, and reverse transcription DNA, etc. Currently, there are also many anticancer drug candidates undergoing clinical trials in this form such as doxorubicin and paclitaxel, and these studies prove that micelles have great value as nano-drug delivery systems and provide a solid basis for the development of drugs for micelle nano-drug delivery systems.

Rubiaceae-type cyclopeptides (RAs) are unique to rubiaceae and are commonly found in rubiaceae plants, which are a class of bicyclic homocyclic hexapeptides, and are mainly by one D-type α-alanine, one L-type α-alanine, three L-type N-substituted α-tyrosines, and one other L-type encoded α-amino acid connected by peptide chains to form a cyclic hexapeptide, and six amino acids condensed into an eighteen-membered ring, in which the phenyl ring between two adjacent tyrosines is connected via an oxygen bridge to form a fourteen-membered ring with greater tension. RAs have attracted much attention because of their novel bicyclic structure and remarkable antitumor activity in vitro and in vivo. Our previous research showed that RAs could inhibit the kinase activity of TAK1 (Chinese invention patent number: CN201410445325.0). In the prior art, no rubiaceae-type cyclopeptides have been shown to inhibit KRAS mutations, especially protective autophagy of KRAS dependent tumor cells promotes its apoptosis, and the preparation method and use of the nano-micelle injection as an antitumor drug.

The Chinese patent application no. CN104262465A describes the preparation of rubiaceae cyclopeptides for use as a TAK1 inhibitors in preparing anti-inflammatory drugs.

The scientific publication entitled "RA-XII inhibits tumour growth and metastasis in breast tumour-bearing mice via reducing cell adhesion and invasion and promoting matrix degradation" (Hoi-Wing Leung et al., Scientific Reports, 2015, vol. 5, no. 1, pgs 1 - 17) describes a study carried out to investigate the *in vitro* effects of cyclopeptide glycoside, RA-XIII on cell adhesion, invasion, proliferation and matrix degradation.

The scientific publication entitled "Polymeric micelles in anticancer therapy: targeting, imaging and triggered release" (Oerlemans et al., Pharm Res, 2010, vol. 27, pgs 2569-2589) is a review of the use of polymeric micelles in cancer therapy.

The scientific publication entitled "Studies on the antitumor cyclic hexapeptides obtained from rubiae racix" (Itokawa et al., Chem. Pharm. Bull, 1983, vol. 31(4), pgs 1424 - 1427) describes methanolic extracts prepared from the roots of Rubia cordifolia L (Rubiaceae) showing antitumor activity in mice.

The scientific publication entitled "Rubiyunnanins C-H, cytotoxic cyclic hexapeptides from Rubia yunnanensis inhibiting nitric oxide production and NF-κB activation" (Jun-Ting Fan et al., 2010, Bioorganic & Medicinal Chemistry, vol. 18, pgs 8226 - 8234)) describes cyclic hexapeptides isolated from the roots of *Rubia yunnanensis* demonstrating cytotoxic activites against cancer cell lines.

Chinese patent application no. CN103877562A describes the application of rubiaceae-type cyclopeptides in the preparation of medicine for treating and preventing cancers activated by the abnormity of Hedgehog signal channels.

### Summary

The invention is defined in the appended claims. In order to solve the gaps in the prior art, the objective of the present invention is to provide a cell autophagy inhibitor using a rubiaceae-type cyclopeptide compound as the active ingredient, and is particularly suitable for inhibiting KRAS mutations, especially KRAS dependent protective autophagy of tumor cells, and promoting tumor cell apoptosis, and the present invention also provides a nano-micelle injection of the rubiaceae-type cyclopeptide compound and a preparation method and a use thereof.

Technical solution: the cell autophagy inhibitor according to the present invention, wherein its active ingredient is a rubiaceae-type cyclopeptide.

Further, the inhibitor is particularly suitable for inhibiting KRAS mutations, especially KRAS dependent protective autophagy of tumor cells, and promoting tumor cell apoptosis.

Specifically, the application takes KRAS dependent tumor cells as the subject, treat the cultured tumor cells with RAs, uses the Western blot method to detect autophagy-related proteins, uses the GFP-LC3 transfection to detect the formation of autophagic vacuoles, and measures the degree of cell death and confirms that the compound inhibits protective autophagy of cells; takes KRAS dependent tumor cells as the subject, treats the cultured tumor cells with RAs, uses the Western blot method to detect autophagy-related proteins, uses the PI/Annexin V double staining to detect the proportion of apoptotic cells, and confirms that the compound induces cell apoptosis. KRAS mutant HCT116 xenograft model and KRAS dependent H441 xenograft model are selected to evaluate the antitumor activity of RAs in vivo.

Further, the rubiaceae-type cyclopeptide is RA-V (1) or RA-XII (2) represented by the following structural formula.

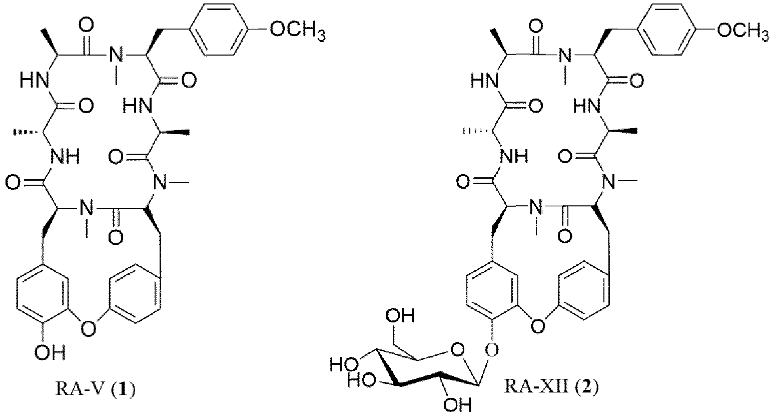

The inhibitor may be in any pharmacologically acceptable formulation, and the dosage thereof is any pharmacologically acceptable dosage.

The inhibitor is a nano-micelle injection. The preparation method of the nano-micelle injection is using a rubiaceae-type cyclopeptide as the active ingredient and a block copolymer as the carrier to form block copolymer micelles, which can significantly improve the solubility and bioavailability of the active ingredient, improve its pharmacokinetic properties, and improve its efficacy.

Specifically, the preparation method of the nano-micelle injection includes the following steps: dissolving the active ingredient and the mPEG2000-PDLLA2000 block copolymer in an organic solvent, shaking it to dissolve and mix thoroughly, and removing the organic solvent by vacuum rotary evaporation to obtain the mixed drug film, and then adding water for injection, shaking to fully dissolve the drug film to obtain a micellar solution; filtering sterilization with a microporous filter, freeze-drying, and packing under sterile conditions to prepare a nano-micelle injection.

In the present invention, the micelle is one of the colloidal dispersion systems, and belongs to an association colloid, is a colloid-sized aggregate nanoparticle automatically associated by the molecule or ion of the amphiphilic block polymer when its concentration in the solution exceeds a certain threshold value, which is used for the solubilization of drugs and can also be used as the carrier of drug delivery systems to improve stability, enhance efficacy, and reduce toxicity. The polymer micelle preparation of the present invention can significantly improve the water solubility of the active ingredient, and utilizes the permeability and retention effect (EPR effect) of polymer micelles on tumor blood vessels, so that nano-micelles are passively targeted and concentrated in tumor tissues to improve the effect of anti-tumor therapy and reduce the drug side effects.

Preferably, the particle size of the prepared nano-micelle injection is 10-100 nm.

Preferably, the effective content of the mPEG2000-PDLLA2000 diblock copolymer of the prepared nano-micelle injection is 1-10% by weight.

The use of the above inhibitors for the preparation of a medicament for the treatment and prevention of diseases that can benefit from the inhibition of cellular autophagy is also within the scope of the present invention. Such uses include, but are not limited to, uses that benefit from diseases that inhibit the protective autophagy of tumor cells.

The inhibitors of the present invention may also be used with other drugs to provide a combination therapy, wherein the other drugs can be combined with the above mentioned inhibitors to form a composition preparation or may be provided as separate compositions for simultaneous or different administration.

The preparation method of the above mentioned rubiaceae-type cyclopeptide is disclosed in the Chinese invention patent CN 201410445325 .0.

Beneficial effect: the cell autophagy inhibitor according to the present invention can effectively inhibit cell autophagy, and the active ingredient rubiaceae-type cyclopeptides have a wide range of sources, mature extraction processes, diversified dosage forms and medication methods, and can be used to the treatment and prevention of KRAS-related cancers and have broad clinical application prospects.

### Brief Description of the Drawings

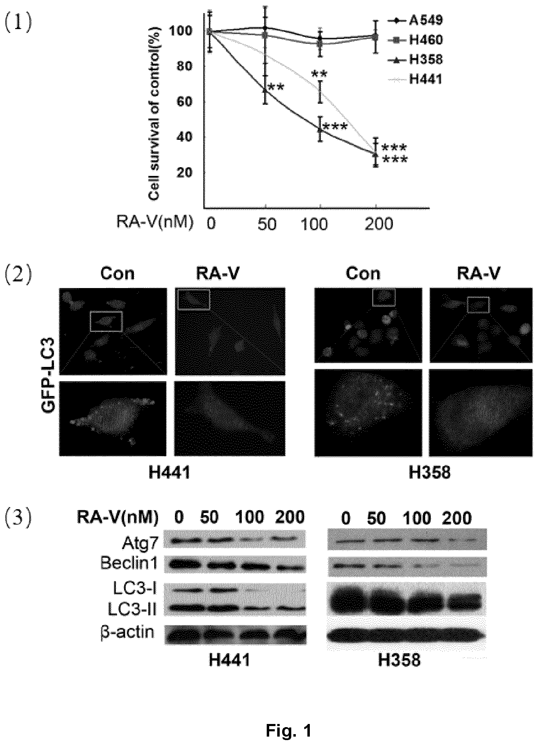

Figure 1 shows that the rubiaceae-type cyclopeptide RA-V (1) inhibits KRAS dependent protective autophagy of tumor cells, in which (1): RA-V (1) inhibits the proliferation of KRAS dependent lung cancer cells H441 and H358; (2): RA-V (1) inhibits the formation of GFP-LC3 autophagosomes of KRAS dependent lung cancer cells H441 and H358; (3): RA-V (1) inhibits expression of the autophagy-related proteins of KRAS dependent lung cancer cells;

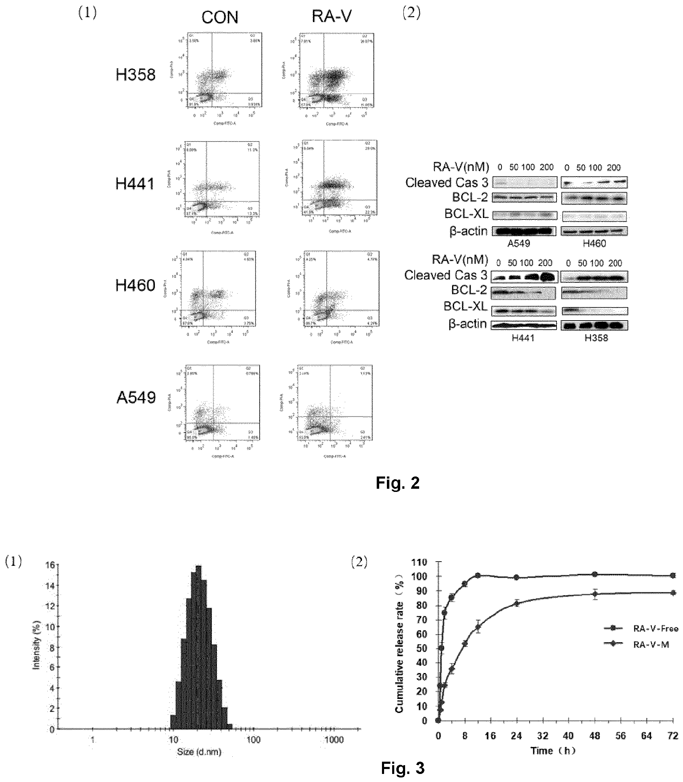

Figure 2 shows that the rubiaceae-type cyclopeptide RA-V (1) induces apoptosis of KRAS dependent tumor cells, wherein (1): Annxin-V/PI staining detection reveals that RA-V (1) promotes apoptosis of KRAS dependent lung cancer cells; (2): Western blot detection reveals that RA-V (1) induces apoptosis of KRAS dependent lung cancer cells;
Figure 3 shows the characterization of nano-micelles of the rubiaceae-type cyclopeptide RA-V (1), wherein (1): the particle size of the micelles; (2): the drug release curve;

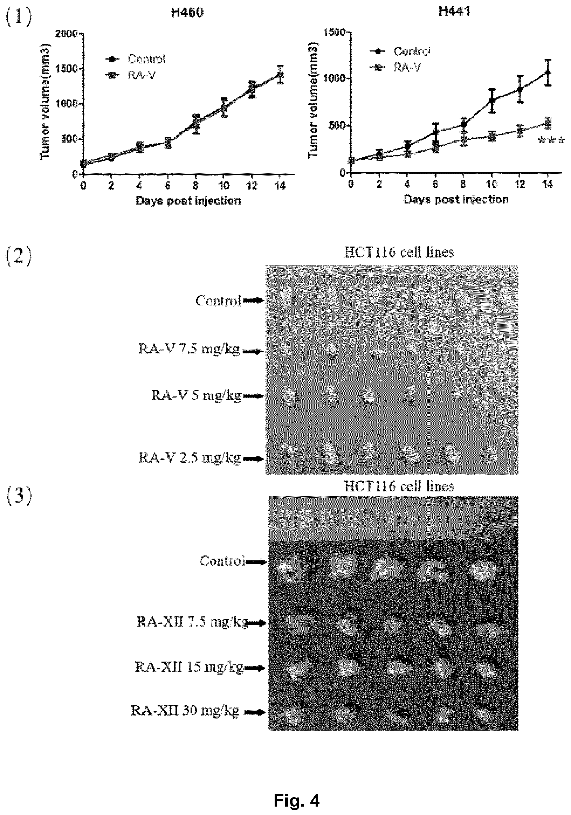

Figure 4 shows the in vivo anti-tumor test of the nano-micelle injection of the rubiaceae-type cyclopeptides RA-V (1) and RA-XII (2), wherein (1): RA-V (1) has better solid tumor suppression effect on KRAS dependent lung cancer cells; (2): the anti-tumor effect of RA-V (1) on KRAS mutant HCT116 model; (3): the anti-tumor effect of RA-XII (2) on KRAS mutant HCT116 model.

### Detailed Description

The following describes the essential content of the present invention with specific embodiments in conjunction with the accompanying drawings, but the present invention is not limited thereto. Preparation of the rubiaceae-type cyclopeptides RA-V (1) and RA-XII (2) refers to patent CN201410445325.0.

### Embodiment 1

RA-V (1) inhibits protective autophagy of KRAS dependent tumor cells:
MTT assay is used to determine the cell viability. KRAS independent A549 and H460 and KRAS dependent H441 and H358 cell lines which are cultured overnight in a 10% FBS medium, are digested by trypsin to form cell suspensions, which are seeded at a suitable concentration on a 96-well plate with 100 µl/well, cultured in a CO2 incubator for 24 hours until the cells are completely attached, and RA-V (1) with a final concentration of 0, 50, 100, 200 nM is added to react for 24 hours, and then 20 µl of MTT solution (5 mg/ml in PBS, pH = 7.4) is added to each well and continue to incubate for 4 hours, stop the culture, and carefully discard the culture supernatant in the wells. Add 150 µl DMSO to each well and shake for 10 minutes to fully dissolve the crystals. Select the wavelength of 490 nm, measure the light absorption value of each well on the enzyme-linked immunosorbent detector, record the results, draw the cell growth curve with time as the abscissa and absorbance value as the ordinate.

KRAS dependent H441 and H358 cells which are cultured overnight in a 10% FBS medium are seeded in a 24-well plate at an appropriate density, after 24 hours, RA-V (1) with a concentration of 0, 50, 100, and 200 nM is added to react for 24 hours, and then the cells are collected to detect the expression levels of autophagy-related proteins LC3, Atg7 and Beclin1 by the Western blot experiment.

KRAS dependent H441 and H358 cells which are cultured overnight in a 10% FBS medium are seeded in a 24-well plate at an appropriate density and transfected with the GFP-LC3 plasmid, after 24 hours, RA-V (1) is added to react for 24 hours, and the localization of GFP-LC3 is observed with a fluorescence microscope, and the number of autophagy cells is calculated quantitatively.

The test results are shown in Figure 1, which show that RA-V (1) inhibits the survival of KRAS dependent lung cancer cells, LC3-II, Atg7, and Beclin1 decrease with the increase of the concentration of RA-V (1), compared with the blank group, the GFP-LC3 of the RA-V (1) treated cells significantly reduce, confirming that RA-V (1) significantly inhibits the protective autophagy of H441 and H358.

### Embodiment 2

RA-V (1) significantly induces KRAS dependent tumor cell apoptosis:
KRAS independent A549 and H460 and KRAS dependent H441 and H358 cell lines which are cultured overnight in a 10% FBS medium, are digested by trypsin to form cell suspensions, which are seeded at a suitable concentration on a 6-well plate, after 24 h, RA-V (1) is added to treat for 24 hours, and are digested by trypsin, and centrifuge at 2000 rpm for 5-10 minutes at room temperature to collect cells; resuspend the cells once with pre-chilled 1×PBS (4°C), centrifuge at 2000 rpm for 5-10 minutes, and wash the cells; add 300 µL of 1×Binding Buffer to resuspend the cells; add 5 µL of Annexin V-FITC and mix, and incubate for 15 minutes at room temperature away from light; add 5 µL of PI staining 5 minutes before the operation, and detect the cells by the flow cytometry.

KRAS dependent A549 and H460 cells which are cultured overnight in a 10% FBS medium are seeded in a 24-well plate at an appropriate density, after 24 hours, the cells are collected to detect the expression levels of autophagy-related proteins BCL-2, BCL-XL and Capsase3 by the Western blot experiment.

The test results are shown in Figure 1, which show that PI/Annexin V double staining indicates that RA-V (1) significantly induces the cell apoptosis of H358 and H441, but has no significant effect on H460 and A549. Detection of the expression of related apoptosis protein Capsase3, and apoptosis inhibitory proteins BCL-2 and BCL-XL also proves that RA-V significantly induces the cell apoptosis of H358 and H441, and has no significant effect on H460 and A549.

### Embodiment 3

Preparation method of mPEG2000-PDLLA2000 micelles:
(1) Dissolution: prepare drug-loaded nano-micelles at a weight ratio of mPEG2000-PDLLA2000 and RA-V (1) of 30-10: 1, accurately weigh mPEG2000-PDLLA2000 and RA-V (1) according to Table 1, and mix and dissolve them in a pear-shaped bottle containing 100 mL of dichloromethane, and shake continuously until the drugs and materials are dissolved. Then add 25 mL of methanol and continue shaking until the drugs and materials are completely dissolved, and a clear solution is obtained after about 5 minutes.
(2) Distilling off the solvent: put the pear-shaped bottle on a rotary evaporator, vacuum spin, rotate at 100 rpm, control the temperature at 60 °C, remove the organic solvent, and after it is removed, reduce the temperature to 40 °C, and continue rotary evaporation under vacuum for 3 hours to remove the remaining organic solvents, to obtain a transparent gel-like mixed drug film of RA-V (1) and mPEG2000-PDLLA2000.
(3) Redissolution: add 25 mL of 0.9% sodium chloride water for injection which is pre-heated at 60°C, quickly shake, and fully dissolve the gel-like drug film to obtain a RA-V (1) nano-micelle solution .
(4) Filtration sterilization: the RA-V (1) nano-micelle solution is filtered through a 0.22 µm microporous filter to sterilize.
(5) Divide and freeze-dry to obtain a RA-V (1) nano-micelle lyophilized powder.
(6) Roll the cap to obtain a finished RA-V (1) nano-micelle injection.

### Embodiment 4

Characterization of RA-V (1) nano-micelles:
(1) Particle size test of RA-V micelle: the micelle solution prepared in Embodiment 3 is diluted 50 times with water, and the particle size of the micelles is measured with a Malvern particle size analyzer, and the results are shown in Table 1:

**Table 1 detection of the particle size of the micelles**

| Embodiments | Particle size /nm | PDI |
|---|---|---|
| RA-V (**1**): mPEG200-PDLLA2000=1: 10 | 25.6 ± 0.8 | 0.28 ± 0.02 |
| RA-V (**1**): mPEG200-PDLLA2000=1 :20 | 22.9 ± 0.4 | 0.18 ± 0.02 |
| RA-V (**1**): mPEG200-PDLLA2000=1 :30 | 19.9 ± 0.2 | 0.16 ± 0.02 |

According to the characterization in Figure 3, it can be seen that the particle size of micelles is generally 10-100 nm, and the particle sizes of the micelles prepared by the present invention are all in the range, which indicates that the process of the present invention is feasible.
(2) Detection of encapsulation rate: the nano-micelle for injection prepared in Embodiment 3 is dissolved with 1 mL of water, and take 50µL of the solution and add 950 µL of acetonitrile, mix by shaking, centrifuge for 3 minutes, take the supernatant through a 0.45 µm microporous membrane, and measure the content of RA-V (1) by HPLC so as to calculate the encapsulation rate of the micelle prepared by copolymer and RA-V (1) to evaluate its solubilizing effect. The results are as follows:

**Table 2 experimental results of encapsulation rate of drug-loaded micelles**

| Embodiments | EE (%) |
|---|---|
| RA-V (**1**): mPEG200-PDLLA2000=1: 10 | 90.8 ± 0.6 |
| RA-V (**1**): mPEG200-PDLLA2000=1 :20 | 91.1 ± 0.9 |
| RA-V (**1**): mPEG200-PDLLA2000=1 :30 | 96.1 ± 0.8 |

The detection results of the nano-micelle content show that the copolymer of the present invention has a strong drug loading capacity for RA-V (1), and more than 90% of RA-V (1) is made into nano-micelles, which fully embody the solubilizing effect of the method of the present invention.
(3) Detection of RA-V (1) in vitro release ability by nano-micelles: place the RA-V (1) nano-micelles prepared in Embodiment 3 or equivalent RA-V (1) powder in a dialysis bag for dialysis with a dialysis medium of 0.5% SDS in PBS solution, at each time point, 1 mL of dialysate is taken out and 1 mL of dialysate is added, and the content of RA-V (1) is measured by HPLC. The RA-V (1) content measured during each fluid change is accumulated and plotted against time, and the release curve of micelles to RA-V (1) can be obtained as shown in Figure 3. The results show that the release of RA-V (1) nano-micelles reaches 89%, indicating that the release performance of RA-V (1) nano-micelles prepared in Embodiment 3 is good.

### Embodiment 5

### (1) Anti-tumor effect of RA-V (1) nano-micelle injection on human lung cancer xenografts in vivo

Human lung cancer cells KRAS dependent H441 and KRAS independent H460 are diluted with physiological saline to 1×107 cells/mL, and 100 µL of the cell suspension is inoculated subcutaneously in the left armpit of BABL/c nude mice and grown for 7 days to form tumor-bearing mouse model. The tumor-bearing mice with good growth are inoculated and randomly divided into groups. The RA-V nano-micelle injection prepared in Embodiment 3 is administered through the tail vein and administered once every other day, and the animals used are sacrificed 14 days after the administration, and the tumors are removed and weighed, calculate tumor suppression rate and perform statistical processing. Tumor inhibition rate (%) = (average tumor weight in the control group-average tumor weight in the experimental group)/average tumor weight in the control group × 100%. The test results are shown in Figure 4, which show that 2.5 mg/kg of RA-V (1) is not effective on the KRAS independent H460 model, but is effective on the KRAS dependent H441 model, and the tumor inhibition rate is 50.4%.

### (2) Anti-tumor effect of RA-V (1) nano-micelle injection on human colon cancer KRAS mutant HCT116 nude mice xenograft in vivo:

HCT116 is diluted with serum-free McCoy's 5a medium to 1×107 cells/mL, and 100 µL of the cell suspension is inoculated subcutaneously in the left armpit of BABL/c nude mice and grown for 7 days to form a tumor-bearing mouse model. The tumor-bearing mice with good growth are inoculated and randomly divided into groups. The RA-V nano-micelle injection prepared in Embodiment 3 is administered through the tail vein and administered once every other day, and the animals used are sacrificed 14 days after the administration, and the tumors are removed and weighed, calculate tumor suppression rate and perform statistical processing. Tumor inhibition rate (%) = (average tumor weight in the control group-average tumor weight in the experimental group)/average tumor weight in the control group × 100%. The test results are shown in Figure 4, which show that the tumor inhibition rates of high and medium dosages of RA-V (1) are 66.67% and 41.67%.

### (3) Anti-tumor effect of RA-XII (2) nano-micelle injection on human colon cancer KRAS mutant HCT116 nude mice xenograft in vivo:

HCT116 is diluted with serum-free McCoy's 5a medium to 1×107 cells/mL, and 100 µL of the cell suspension is inoculated subcutaneously in the left armpit of BABL/c nude mice and grown for 7 days to form a tumor-bearing mouse model. The tumor-bearing mice with good growth are inoculated and randomly divided into groups. RA-XII(2) injection is administered through the tail vein and administered once every other day, and the animals used are sacrificed 14 days after the administration, and the tumors are removed and weighed, calculate tumor suppression rate and perform statistical processing. Tumor inhibition rate (%) = (average tumor weight in the control group-average tumor weight in the experimental group)/average tumor weight in the control group × 100%. The test results are shown in Figure 4, which show that the tumor inhibition rates of high and medium dosages of RA-XII(2) are 79.92% and 68.47%.

## Claims

1. A protective cell autophagy inhibitor, wherein its active ingredient is a rubiaceae-type cyclopeptide, wherein the active ingredient is a rubiaceae-type cyclopeptide RA-V (1) or RA-XII (2) represented by the following structural formula: 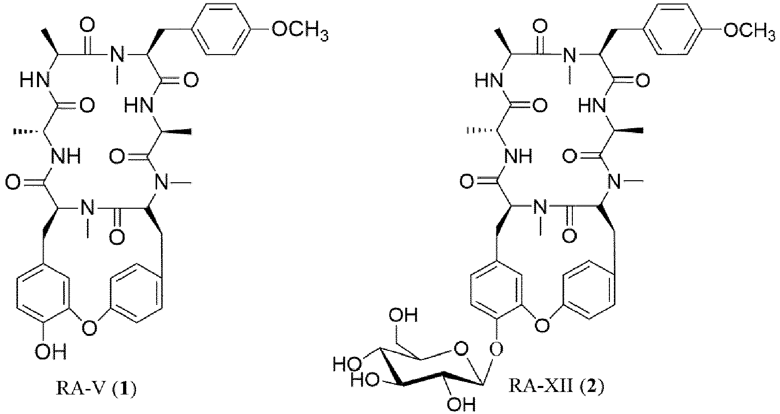 , for use in the treatment and prevention of tumors with KRAS gene mutations, and wherein the inhibitor is a nano-micelle injection.

2. The inhibitor for use according to claim 1, wherein the nano-micelle injection is prepared using a rubiaceae-type cyclopeptide as the active ingredient and a block copolymer as the carrier.

3. The inhibitor for use according to claim 2, wherein the carrier is an mPEG2000-PDLLA2000 amphiphilic block copolymer.

4. The inhibitor for use according to claim 2, wherein the particle size of the nano-micelle injection is 10-100 nm.

5. The inhibitor for use according to claim 2, wherein the block copolymer is a mPEG2000-PDLLA2000 diblock copolymer and wherein the effective content of the mPEG2000-PDLLA2000 diblock copolymer of the nano-micelle injection is 1-10%.

6. The inhibitor for use according to claim 1, wherein the tumors with KRAS gene mutations are colon cancer, rectal cancer, lung cancer, and pancreatic cancer.

7. The inhibitor for use according to claim 1, wherein the inhibitor induces tumor cell apoptosis in tumors with KRAS gene mutations, especially protective autophagy of KRAS dependent tumor cells.

## Patentansprüche

1. Inhibitor einer schützenden Zellenautophagie, wobei sein Wirkstoff ein Cyclopeptid einer Rubiaceae-Art ist, wobei der Wirkstoff ein Cyclopeptid der Rubiaceae-Art RA-V (1) oder RA-XII (2) dargestellt durch die folgende Strukturformel ist: 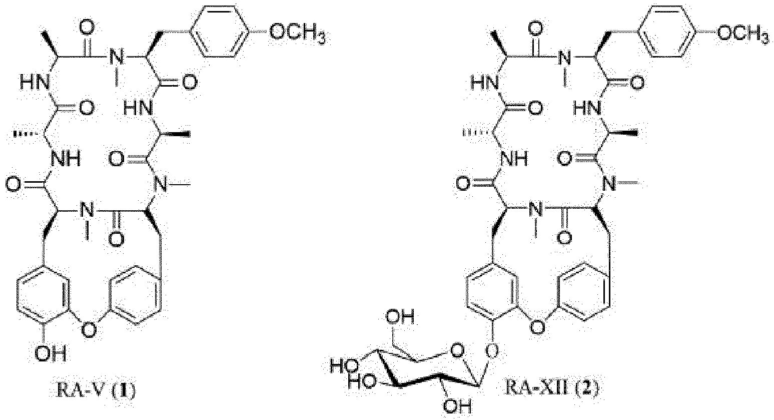 zur Verwendung bei der Behandlung und der Vorbeugung von Tumoren mit KRAS-Genmutationen, und wobei der Inhibitor eine Nanomizelleninjektion ist.

2. Inhibitor zur Verwendung nach Anspruch 1, wobei die Nanomizelleninjektion unter Verwendung eines Cyclopeptids der Rubiaceae-Art als den Wirkstoff und eines Blockcopolymers als den Träger hergestellt wird.

3. Inhibitor zur Verwendung nach Anspruch 2, wobei der Träger ein amphiphiles mPEG2000-PDLLA2000-Blockcopolymer ist.

4. Inhibitor zur Verwendung nach Anspruch 2, wobei die Partikelgröße der Nanomizelleninjektion 10-100 nm beträgt.

5. Inhibitor zur Verwendung nach Anspruch 2, wobei das Blockcopolymer ein mPEG2000-PDLLA2000-Diblockcopolymer ist und wobei der wirksame Gehalt des mPEG2000-PDLLA2000-Diblockcopolymers der Nanomizelleninjektion 1-10 %beträgt.

6. Inhibitor zur Verwendung nach Anspruch 1, wobei die Tumore mit KRAS-Genmutationen Dickdarmkrebs, Rektalkrebs, Lungenkrebs und Bauchspeicheldrüsenkrebs sind.

7. Inhibitor zur Verwendung nach Anspruch 1, wobei der Inhibitor die Tumorzellapoptose in Tumoren mit KRAS-Genmutationen induziert, insbesondere eine schützende Autophagie von KRAS-abhängigen Tumorzellen.

## Revendications

1. Inhibiteur d'autophagie cellulaire protectrice, dans lequel son ingrédient actif est un cyclopeptide de type rubiacées, l'ingrédient actif étant un cyclopeptide de type rubiacées RA-V (1) ou RA-XII (2) représenté par la formule structurale suivante : 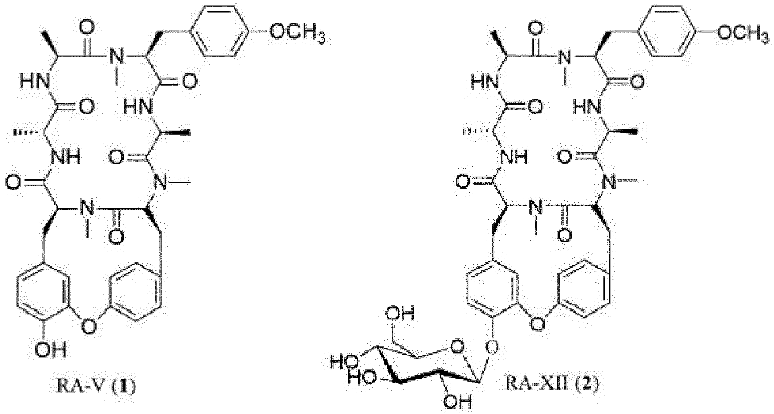 destiné à être utilisé dans le traitement et la prévention de tumeurs présentant des mutations du gène KRAS, et l'inhibiteur étant une injection de nano-micelles.

2. Inhibiteur destiné à être utilisé selon la revendication 1, dans lequel l'injection de nano-micelles est préparée à l'aide d'un cyclopeptide de type rubiacées comme ingrédient actif et un copolymère séquencé comme support.

3. Inhibiteur destiné à être utilisé selon la revendication 2, dans lequel le support est un copolymère séquencé amphiphile mPEG2000-PDLLA2000.

4. Inhibiteur destiné à être utilisé selon la revendication 2, dans lequel la taille des particules de l'injection de nano-micelles est de 10 à 100 nm.

5. Inhibiteur destiné à être utilisé selon la revendication 2, dans lequel le copolymère séquencé est un copolymère biséquencé mPEG2000-PDLLA2000 et la teneur efficace du copolymère biséquencé mPEG2000-PDLLA2000 de l'injection de nano-micelle étant de 1 à 10 %.

6. Inhibiteur destiné à être utilisé selon la revendication 1, dans lequel les tumeurs avec des mutations du gène KRAS sont le cancer du côlon, le cancer du rectum, le cancer du poumon et le cancer du pancréas.

7. Inhibiteur destiné à être utilisé selon la revendication 1, dans lequel l'inhibiteur induit l'apoptose des cellules tumorales dans les tumeurs présentant des mutations du gène KRAS, en particulier l'autophagie protectrice des cellules tumorales dépendantes de KRAS.
